# EUROPEAN PATENT SPECIFICATION

(11) **EP 2 988 687 B1**
(45) Date of publication and mention of the grant of the patent: **09.08.2017**
(21) Application number: 13737429.4
(22) Date of filing: 24.04.2013
(51) Int. Cl.: A61B 17/68

(54) **DEVICE FOR FIXING A CRANIAL LIMB TO THE CRANIAL CROWN TO BE PLACED IN THE CRANIOTOMIAL HOLE OR CUTTING**
VORRICHTUNG ZUR BEFESTIGUNG EINES SCHÄDELGLIEDS AN DER SCHÄDELKRONE ZUR PLATZIERUNG IN EINER SCHÄDELBOHRUNG ODER EINEM SCHNITT
DISPOSITIF DE FIXATION D'UN MEMBRE CRÂNIEN AU SOMMET CRÂNIEN DEVANT ÊTRE PLACÉ DANS L'ORIFICE OU LA COUPE DE CRANIOTOMIE

(43) Date of publication of application: 02.03.2016
(73) Proprietor: Ntplast S.r.l., 15050 Rivalta Scriva (AL) (IT)
(72) Inventor: CARVANI, MORENO, 1-15057 Rivalta Scrivia Tortona (AL) (IT); GAZZANI, IGINO ROMOLO, 1-15057 Rivalta Scrivia Tortona (AL) (IT); CAVIGLIASSO, PIERO, 1-15057 Rivalta Scrivia Tortona (AL) (IT)
(74) Representative: Garavelli, Paolo
(86) International application number: PCT/IT2013/000122
(87) International publication number: WO 2014/174538

(56) References cited:
- WO-A2-2008/002595
- US-A1- 2008 281 339

## Description

The present invention refers to a device for fixing a cranial limb to the cranial crown to be placed in the craniotomial hole or cutting. An applying device dedicated for such device and a process for using such device are also described.

The present invention is an improvement of the device disclosed in document WO-A1-2009/044421 of the same Applicant.

Document US2008281339 A1 discloses a device according to the preamble of claim 1.

As known, craniotomy, namely incision and cutting a bone limb of the cranial crown, is the compulsory neuro-surgical procedure for treating any intra-cranial lesion.

The bone limb is cut by performing one or more drill holes, according to two preferred procedures. The first procedure provides for the use of a cutting blade or perforating device applied to the pneumatic drill, that, with free hand, from the key hole detach the *dura mater* below and simultaneously etches the bone. The second procedure, instead, provides that, with a curved periosteum-detaching device, the *dura mater* is cut from the bone between a drill hole and another, and, afterwards, using a guide, a saw wire is passes that, pulled upwards at its two ends with wide hands, cuts the bone one segment at a time.

At the end of the neuro-surgical intervention, after having sutured the *dura mater* and suspended the edges to the bone, the limb is laid again in the opening and fastened with separate metal or wire staples, made pass through small drill holes coupled on the free edge of the cranial bone.

It is however clear that this type of solution does not allow, in general, an aesthetically acceptable closure, since it is not always able to avoid that the bone limb can be projected, recessed, slanted or rotated.

Object of the present invention, therefore, is solving the above prior art problems, by providing a device for fixing a cranial limb to the cranial crown that allows a correct recovery and attachment of the bone limb to the edge of the cranial crown and simultaneously closes the holes obtained for the craniotomy.

Another object of the invention is providing a device that allows having a lower tension on the brain, even in case a brain edema occurs after the surgery.

With respect to the invention disclosed in the above mentioned patent WO-A1-2009/044421, that necessarily requires the presence of drill holes to be placed, the present invention can be inserted indifferently into the craniotomial hole or into the craniotomial cutting, thereby allowing to fix the limb in many points, even when a single hole has been drilled, or this latter one has an irregular shape. Moreover, an applying device is described, dedicated to simplify the assembly of the device.

Another object of the present invention is making a lower base to be assembled on the above mentioned device to improve its functionality in the following ways:
- increasing the resistance to compression loads that could act on the operculum after the craniotomy closure (ex. accidental impacts to which the patient could be subjected), due to a greater abutment surface;
- allowing the elastic arms of the device to easily slide during the positioning phase, independently from the presence of irregularities in the internal cranial surface; and
- more uniformly distributing the loads onto the internal cranial surface.

As optional feature, the base can be equipped with a centring element from which, when the device is placed inside a hole obtained by the drill, the operculum is better centred in the craniotomy.

These and other objects are obtained by a device for fixing a cranial limb to the cranial crown to be placed into the craniotomial hole or into the craniotomial cutting as described in claim 1.

Further features of the invention are defined in the dependent claims.

It is intended that all claims are an integral part of the present document.

Further objects and advantages of the present invention will be clear from the following description and from the attached drawings, provided merely as a non-limiting example, in which:
- Figure 1 is a top perspective view of a first preferred embodiment of the device for fixing a cranial limb to the cranial crown to be placed into the craniotomial hole or the craniotomial cutting;
- Figure 2 is a top perspective view of a second preferred embodiment of the device for fixing a cranial limb to the cranial crown to be placed into the craniotomial hole or the craniotomial cutting;
- Figure 3 is a bottom perspective view of the device of Figure 1;
- Figure 4 is a bottom perspective view of the device of Figure 2;
- Figure 5 is a view of the device of Figure 1 or 2 in a first step of its use in the craniotomial hole;
- Figure 6 is a view of the device of Figure 1 or 2 in a first step of its use in the craniotomial cutting;
- Figure 7 is a view of the device of Figure 1 or 2 in a second step of its use in the craniotomial hole;
- Figure 8 is a view of the device of Figure 1 or 2 in a second step of its use in the craniotomial cutting;
- Figures 9 and 10 are views of the device of Figure 1 or 2 in the final steps of its use in the craniotomial hole/cutting;
- Figures 11 and 13 are a top view and a bottom view (this latter one that better points out the blocking system of the small elastic arms of the device of Figure 1), respectively, of a first embodiment of the base to be used with the device of Figure 1;
- Figures 12 and 14 are a top view and a bottom view (this latter one that better points out the blocking system of the small elastic arms of the device of Figure 1), respectively, of a second embodiment of the base to be used with the device of Figure 2;
- Figures 15 and 17 are a top view and a bottom view, respectively, of a third embodiment of the device of the present invention; and
- Figures 16 and 18 are a top view and a bottom view, respectively, of a fourth embodiment of the of the device of the present invention.

With particular reference to the cited Figures, the device for fixing a cranial limb to the cranial crown to be placed into the craniotomial hole or into the craniotomial cutting, according to the present invention, is globally designated by reference number 10.

The device 10 has elongated elastic arms 11, joined to the cortical riser 12, and ending in small elongated arms 13 placed transversally with respect to the axis of the elongated elastic arms 11, and an upper plate 14, that will have to be inserted into the upper part of the cortical riser 12.

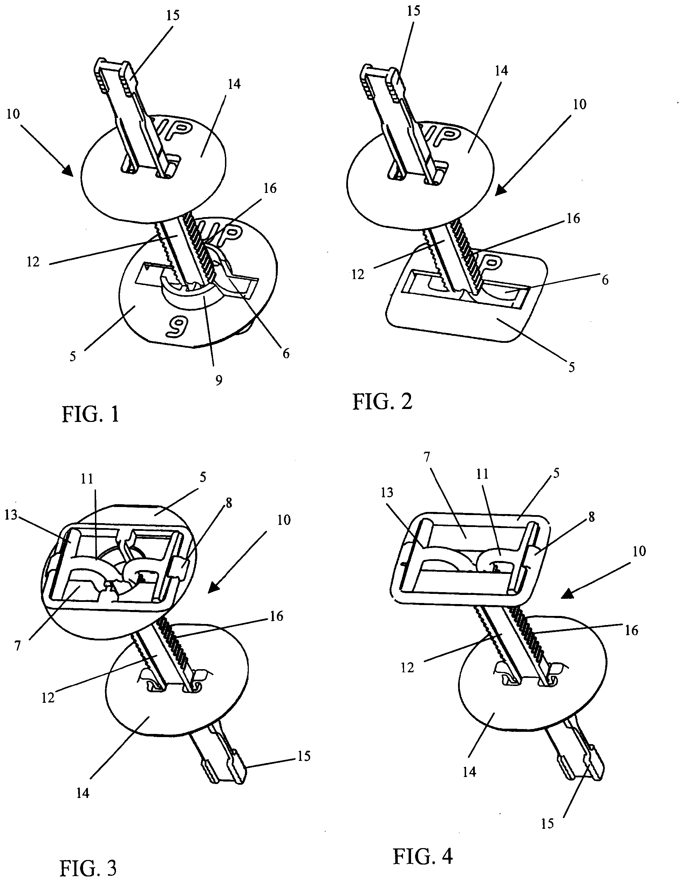

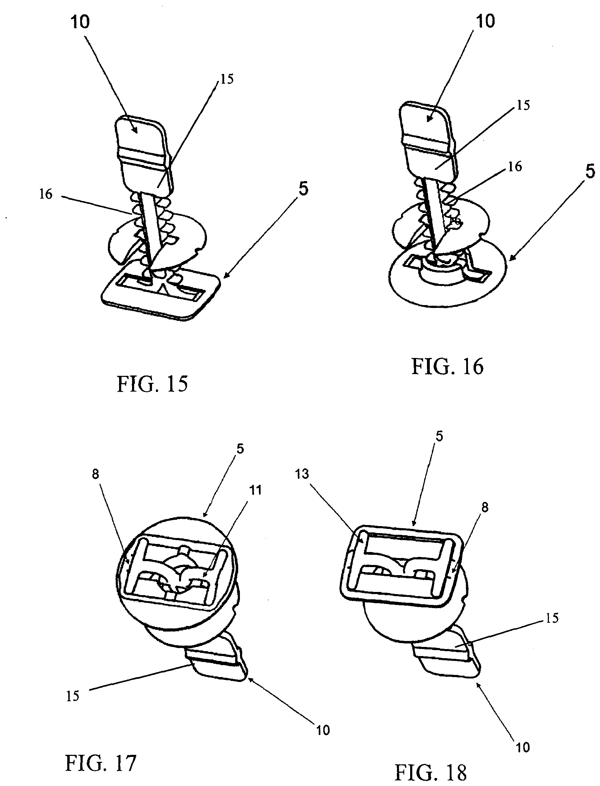

The cortical riser 12 will be equipped with a handle 15 and a plurality of fastening elements 16 (which can be, for example, small teeth as shown in Figures 1 to 9, or transverse projections as shown in Figures 15 to 18) placed at as many levels along the length of the riser 12, fastening elements 16 that will be used for inserting the upper plate 14.

The cortical riser 12, that connects the elastic arms 11 and the upper plate 14, due to the elasticity of the arms 11, allows a fine adjustment to the bone thickness.

The arrangement of the inventive device 10 therefore provides that the first closing means 11, 13 are connected to the distal end of the cortical riser 12 and the second closing means 14 are adapted to be fastened to the cortical riser 12 in a position next to the distal end of the cortical riser 12, in such a way that, when the device 10 is in its operating fastening position, the first closing means 11, 13 are placed against the internal distal cranial surface and the second closing means 14 are placed against the external near cranial surface.

By examining in more detail the application modes of the cranial fastening device 10 of the invention, it must be noted that the instrument operator prepares the right amount of devices 10 suitable for fixing the craniotomy.

Such operations are performed by choosing the more suitable number of devices 10, depending on the width and shape of the craniotomy.

In order to use the device 10 in practice, an applying device (not shown) is preferably, but not exclusively, provided, adapted to operate with the above described device 10; such applying device comprises a seat adapted to support the upper plate 14 and to allow the operating coupling between such plate 14 and the cortical riser 12 when the craniotomial hole or the craniotomial cutting has been closed.

In order to position the device 10 into the craniotomial hole, the following operations are provided.

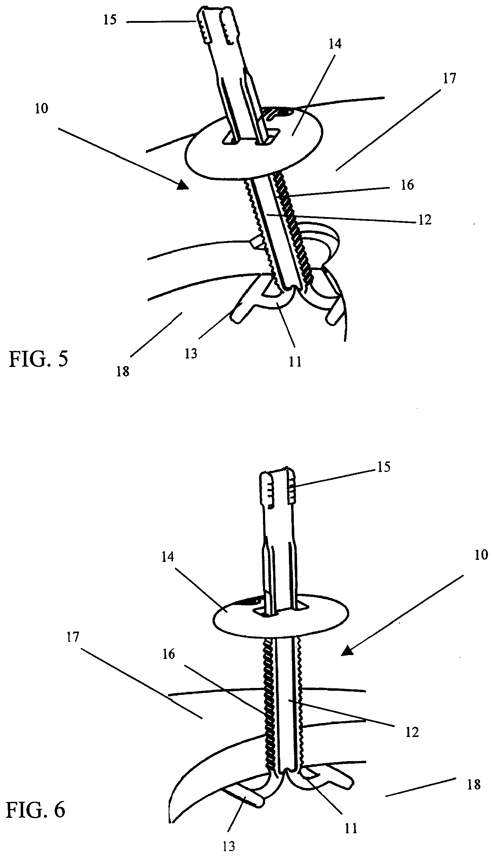

A surgeon inserts the device 10 into the craniotomial hole (Figure 5) with the cortical riser 12 placed at the hole centre and perpendicular to the cranial surface, and so that part of the small cross arms 13 are placed in the space between the cranial crown 17 and the *dura mater* 18.

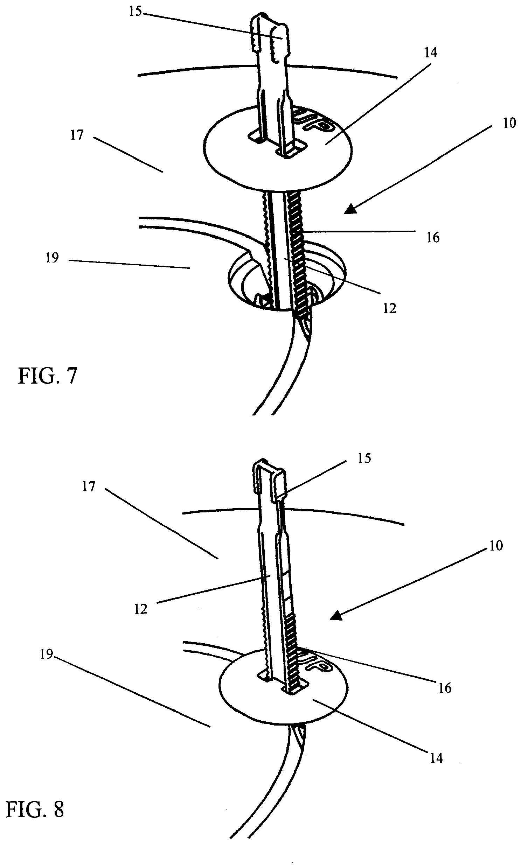

The bone operculum 19 is repositioned as shown in Figure 7. In this way, the cortical riser 12 will project from the craniotomial hole.

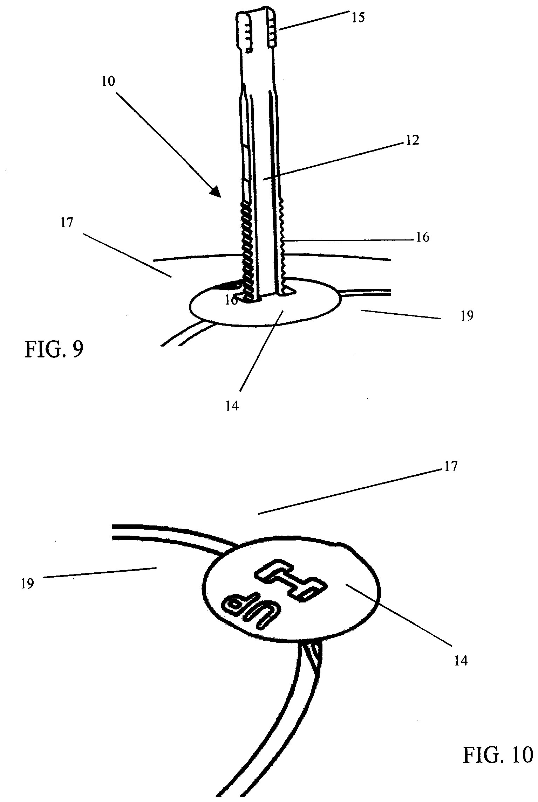

Afterwards, the surgeon verifies the correct position of the operculum 19, and stretches, by means of the handle 15, the cortical riser 12 and the elastic arms 11 in order to be able to insert the upper plate 14 next to one of the fastening levels 16, like in Figure 9. When inserting the upper plate 14, the surgeon uses the above-described applying device. In this way, the craniotomial hole will be closed. Finally, the surgeon removes the handle 15 with a cutting device (Figure 10).

In order to position the device 10 into the craniotomial cutting, the following operations are provided.

A surgeon inserts the device 10 into the craniotomial cutting (Figure 6) so that an end of the small transverse arms 13 is placed in the space between the cranial crown 17 and the *dura mater* 18, with the elastic arms 11 aligned along the direction of the craniotomial cutting and the cortical riser 12 perpendicular to the cranial surface.

The bone operculum 19 is rested onto the other ends of the small transverse arms 13, as shown in Figure 8. In this way, the riser 12 will project between the cranial crown 17 and the operculum 19.

Afterwards, the surgeon verifies the correct position of the operculum 19, and stretches, by means of the handle 15, the cortical riser 12 and the elastic arms 11 to be able to insert the upper plate 14 next to one of the fastening levels 16, like in Figure 9. When inserting the upper plate 14, the surgeon uses the above-described applying device 20. Finally, the surgeon removes the handle 15 with a cutting device (Figure 10).

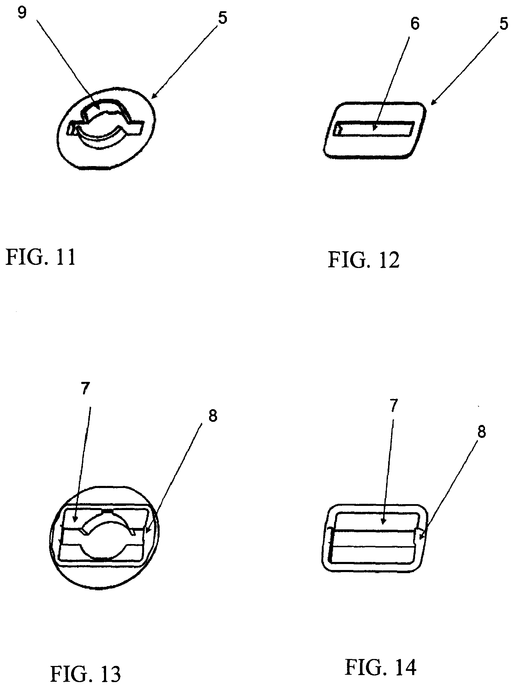

The device 10 of the invention further comprises, as shown in the various Figures and in detail in Figures 11 to 14 in two preferred, but not limiting, configurations thereof, a lower base 5, preferably made of a biocompatible elastic plastic material, to be assembled with the device 10 for fastening a cranial limb and for simultaneously closing the holes obtained for performing the craniotomy.

With particular reference to Figures 11 to 14 actually, the lower base according to the present invention is globally designated by numeric reference 5; the device for fastening a cranial limb to the cranial crown and for simultaneously closing the craniotomy holes, as previously described, is globally designated by numeric reference 10.

The lower base 5 has a cut-out 6 through which the device 10 is passes, and a seat 7 where the small transverse arms 13 linked to the elastic arms 11 are housed and slide. In this way, by sliding the small transverse arms 13 when positioning the device 10, independently from the presence of irregularities in the internal cranial surface, a better application functionality of the device 10 is obtained.

The small transverse arms 13 are blocked, preventing them from being excessively and uselessly opened, by the same shape of the seat 7 of the base 5, or by suitable fastening means 8 provided at two ends of the seat 7 itself (as can be seen in Figures 3, 4, 17 and 18).

A second version of the lower base 5, shown in Figures 1, 11 and 16, has a centring system 9, provided for using the device 10 in holes obtained by the drill.

The cranial fastening device 10 of the invention is suitable for all craniotomy cases, since its shape and its suitability to the bone thickness allow keeping the bone limb tended to the cranial crown edge, both on the internal and on the external margin, in order to allow the correct ossification.

At the same time, the elasticity of the biocompatible plastic material being used guarantees a lower tension on the brain after an operation, namely when brain edema conditions could occur and therefore a swelling of the brain itself.

From the above description, the features are clear for the device 10 for fastening a cranial limb to the cranial crown to be positioned into the craniotomial hole or the craniotomial cutting, object of the present invention, and its advantages are also clear.

Moreover, in the practice of the invention, materials, shapes, sizes of the shown details could be changed according to needs and they could be replaced by other technically equivalent arrangements.

Finally, it is clear that numerous variations could be made to the device 10 for fastening a cranial limb to the cranial crown to be positioned into the craniotomial hole or the craniotomial cutting, object of the present invention, without thereby departing from the scope of the present invention, as defined by the enclosed claims.

## Claims

1. Device (10) for fastening a cranial limb to a cranial crown (17) adapted to be positioned both into a craniotomial hole and into a craniotomial cutting, comprising:
- at least one cortical supporting riser (12);
- first closing means (11, 13) operatively connected to said cortical riser (12);
- second closing means (14) adapted to be fastened to said cortical riser (12) to complete its closing;
- at least one handle (15) removably connected to said cortical riser (12) and adapted to drive said device (10) to take it from its rest position to its operating fastening position;
wherein said first closing means (11, 13) are connected to a distal end of the cortical riser (12) and said second closing means (14) are adapted to be fastened to the cortical riser (12) in a near position of the distal end of the cortical riser (12), so that, when the device (10) is in its operating fastening position, said first closing means (11, 13) are placed against the internal distal cranial surface and said second closing means (14) are placed against the external near cranial surface, said first closing means (11, 13) being composed of at least two elongated elastic arms (11) operatively connected to said cortical riser (12), each one of said elongated elastic arms (11) ending in at least one small elongated arm (13) placed transversally with respect to said elongated elastic arms (11).
**characterised in that** said device (10) is further equipped with at least one lower base (5) to be assembled with the device (10) for fastening a cranial limb and for simultaneously closing holes obtained to perform the craniotomy, said lower base (5) having a cut-out (6) through which said device (10) passes when said device (10) is moved to its operating fastening position, and a seat (7) where said small transverse arms (13) linked to said elastic arms (11) are housed and slide.

2. Device (10) according to claim 1, **characterised in that** said small transverse arms (13) are blocked when opening by a shape of said seat (7) of said base (5).

3. Device (10) according to claim 1, **characterised in that** said small transverse arms (13) are blocked when opening by fastening means (8) provided at two ends of said seat (7) of said base (5).

4. Device (10) according to any one of the previous claims, **characterised in that** said base (5) has a centring system (9) provided to be used when the device (10) is employed in holes obtained with a drill.

5. Device (10) according to any one of the previous claims, **characterised in that** it is composed of elastic material made of biocompatible plastic.

6. Device (10) according to claim 1, **characterised in that** said second closing means (14) are composed of an upper plate (14) adapted to be inserted into the upper part of said cortical riser (12).

7. Device (10) according to claim 6, **characterised in that** said cortical riser (12) is further equipped with a plurality of fastening elements (16) placed at as many levels along a length of the riser (12), said fastening elements (16) being adapted to engage said upper plate (14) after its operating coupling with said riser (12).

## Patentansprüche

1. Vorrichtung (10) zur Befestigung eines Kopflappens an der Schädelkalotte (17), die dazu dient, sowohl in einem Schädelloch als auch in einem Schädelschnitt positioniert zu werden, die Folgendes einschließt:
- mindestens ein kortikales Knochenstück als Halterung (12);
- erste Schließvorrichtungen (11, 13), die operativ mit dem genannten kortikalen Knochenstück (12) verbunden sind;
- zweite Schließvorrichtungen (14), die dazu dienen, mit dem genannten kortikalen Knochenstück (12) verbunden zu werden, um seine Schließung zu vollenden;
- mindestens ein Griff (15), der abnehmbar mit dem genannten kortikalen Knochenstück (12) verbunden ist und dazu dient, die genannte Vorrichtung (10) zu bewegen, um sie aus ihrer Ruhestellung in ihre operative Befestigungsposition zu führen;
in der die genannten Schließvorrichtungen (11, 13) mit dem distalen Ende des kortikalen Knochenstücks (12) verbunden sind und die genannten zweiten Schließvorrichtungen (14) dazu dienen, am kortikalen Knochenstück (12) in einer Position nahe am distalen Ende des kortikalen Knochenstücks (12) befestigt zu werden, sodass die genannten ersten Schließvorrichtungen (11, 13), wenn sich die Vorrichtung (10) in der operativen Befestigungsposition befindet, an der internen distalen Oberfläche des Schädels angebracht sind, und die genannten zweiten Schließvorrichtungen (14) an der externen Oberfläche nahe am Schädel angebracht sind, die genannten ersten Schließvorrichtungen (11, 13) bestehen aus mindestens zwei länglichen elastischen Armen (11), die operativ mit dem genannten kortikalen Knochenstück (12) verbunden sind, jeder der genannten länglichen elastischen Arme (11) endet in mindestens einem länglichen Arm (13), der transversal zu den genannten länglichen elastischen Armen (11) angebracht ist,
die **dadurch gekennzeichnet ist, dass** die genannte Vorrichtung (10) außerdem mit mindestens einer unteren Basis (5) ausgestattet ist, die mit der Vorrichtung (10) für die Befestigung eines Kopflappens und für die gleichzeitige Schließung der Bohrlöcher zusammengebaut werden muss, die für die Ausführung der Kraniotomie angebracht wurden, die genannte untere Basis (5) hat einen Schnitt (6), den die genannte Vorrichtung (10) durchquert, wenn sich die genannte Vorrichtung (10) in ihrer operativen Befestigungsposition bewegt, und eine Aufnahme (7), wo die genannten Schrägarme (13) sitzen und gleiten, die mit den genannten elastischen Armen (11) verbunden sind.

2. Vorrichtung (10) gemäß Patentanspruch 1, die **dadurch gekennzeichnet ist, dass** die genannten Schrägarme (13) bei der Öffnung durch die Form der genannten Aufnahme (7) der genannten Basis (5) blockiert sind.

3. Vorrichtung (10) gemäß Patentanspruch 1, die **dadurch gekennzeichnet ist, dass** die genannten Schrägarme (13) bei der Öffnung durch Befestigungsvorrichtungen (8), die an den beiden Enden der genannten Aufnahme (7) der genannten Basis (5) vorgesehen sind, blockiert sind.

4. Vorrichtung (10) gemäß einem der vorhergehenden Patentansprüche, die **dadurch gekennzeichnet ist, dass** die genannte Basis (5) ein Zentriersystem (9) hat, das für die Anwendung vorgesehen ist, wenn die Vorrichtung (10) in Bohrlöchern angewendet wird, die durch einen Bohrer angebracht wurden.

5. Vorrichtung (10) gemäß einem der vorhergehenden Patentansprüche, die **dadurch gekennzeichnet ist, dass** sie aus elastischem Material aus biokompatiblem Kunststoff hergestellt ist.

6. Vorrichtung (10) gemäß Patentanspruch 1, die **dadurch gekennzeichnet ist, dass** die genannten zweiten Schließvorrichtungen (14) aus einer oberen Platte (14) bestehen, die dazu dient, in den oberen Teil des genannten kortikalen Knochenstücks (12) eingesetzt zu werden.

7. Vorrichtung (10) gemäß Patentanspruch 6, die **dadurch gekennzeichnet ist, dass** das genannte kortikale Knochenstück (12) außerdem mit mehreren Befestigungselementen (16) ausgestattet ist, die auf ebenso vielen Ebenen an der Länge des Knochenstücks (12) angebracht sind, die genannten Befestigungselemente (16) dienen dazu, die genannte obere Platte (14) nach ihrer operativen Verbindung mit dem genannten Knochenstück (12) zu spannen.

## Revendications

1. Dispositif (10) pour la fixation d'une bande crânienne à une voûte crânienne (17) prédisposée pour être positionnée soit dans un trou craniotomique soit dans une coupe craniotomique, comprenant :
- au moins un montant cortical de support (12) ;
- des premiers moyens de fermeture (11, 13) reliés opérationnellement au montant cortical (12) ;
- des seconds moyens de fermeture (14) prédisposés pour la fixation au montant cortical (12) pour compléter sa fermeture ;
- au moins une poignée (15) reliée de manière amovible au montant cortical (12) servant à manoeuvrer le dispositif (10) pour le déplacer de sa position de repos à sa position opérationnelle de fixation ;
où les premiers moyens de fermeture (11, 13) sont reliés à l'extrémité distale du montant cortical (12) et les seconds moyens de fermeture (14) sont prédisposés pour être fixés au montant cortical (12) dans une position proche de l'extrémité distale du montant cortical (12) de sorte que, quand le dispositif (10) se trouve en position opérationnelle de fixation, les premiers moyens de fermeture (11, 13) sont placés contre la surface distale interne du crâne et les seconds moyens de fermeture (14) sont placés contre la surface proche externe du crâne, les premiers moyens de fermeture (11,13) sont composés au moins de deux bras élastiques allongés (11) reliés opérationnellement au montant cortical (12), chaque bras élastique allongé (11) se termine au moins par une patte allongée (13) placée transversalement par rapport aux bras élastiques allongés (11).
**caractérisé en ce que** le dispositif (10) est doté en outre au moins d'une base inférieure (5) à assembler au dispositif (10) pour la fixation d'une bande crânienne et la fermeture simultanée des trous percés pour exécuter la craniotomie ; la base inférieure (5) possède une coupe (6) à travers laquelle passe le dispositif (10) quand il se déplace en position opérationnelle de fixation et un logement (7) qui accueille et où glissent les pattes transversales (13) reliées aux bras élastiques (11).

2. Dispositif (10), selon la revendication 1, **caractérisé en ce que** l'ouverture des pattes transversales (13) est bloquée par la conformation du logement (7) de la base (5).

3. Dispositif (10), selon la revendication 1, **caractérisé en ce que** l'ouverture des pattes transversales (13) est bloquée par des moyens d'accrochage (8) prévus aux deux extrémités du logement (7) de la base (5).

4. Dispositif (10) selon l'une des revendications précédentes, **caractérisé en ce que** la base (5) est dotée d'un système de centrage (9) prévu pour l'utilisation quand le dispositif (10) est engagé dans les trous percés par une perceuse.

5. Dispositif (10) selon l'une des revendications précédentes, **caractérisé en ce qu'**il est en matière élastique et en plastique biocompatible.

6. Dispositif (10), selon la revendication 1, **caractérisé en ce que** les seconds moyens de fermeture (14) sont composés d'un plateau supérieur (14) apte à être introduit dans la partie supérieure du montant cortical (12).

7. Dispositif (10), selon la revendication 6, **caractérisé en ce que** le montant cortical (12) est doté en outre d'une pluralité d'éléments de fixation (16) situés à autant de niveaux tout au long de la longueur du montant (12) ; ces éléments de fixation (16) sont aptes à engager le plateau supérieur (14) après son accouplement opérationnel au montant (12).
